# EUROPEAN PATENT APPLICATION

(11) **EP 1 568 445 A1**
(43) Date of publication of application: **31.08.2005**
(21) Application number: 03756651.0
(22) Date of filing: 16.10.2003
(51) Int. Cl.: B25J 9/06, B25J 7/00, A61B 19/00

(54) **POSITIONING UNIT, AND POSITIONING ARM USING THE SAME**

(30) Priority: 21.10.2002 JP 2002305466
(71) Applicant: THK CO., LTD., Tokyo (JP); Sakuma, Ichiro, Kanagawa 240-0045 (JP); Masamune, Etsuko, Tokyo 174-0071 (JP); Dohi, Takeyoshi, Tokyo 158-0091 (JP)
(72) Inventor: DOHI, Takeyoshi, Setagaya-ku, Tokyo 158-0091 (JP); SAKUMA, Ichiro, Yokohama-shi, Kanagawa 240-0045 (JP); MASAMUNE, Etsuko, Itabashi-ku, Tokyo 174-0071 (JP); KIM, Daeyoung, Nakano-ku, Tokyo 164-0012 (JP); NAKAZAWA, Toji, c/o THK Co., Ltd., Shinagawa-ku, Tokyo 141-8503 (JP)
(74) Representative: Grünecker, Kinkeldey, Stockmair & Schwanhäusser Anwaltssozietät
(86) International application number: PCT/JP2003/013249
(87) International publication number: WO 2004/035270

(57) **Abstract**

Provided is a unit used in a surgical operation using an endoscope, for example, to position the distal end of a surgical instrument to the region of a human body where medical treatment is required, without spreading a hole formed in the human body for insertion of the surgical instrument. This unit includes first and second output shafts pivotally connected to a movable member of the surgical instrument or the like, the first and second output shafts extending parallel to each other and being capable of reciprocating, and a drive unit for giving the first output shaft an arbitrary amount of advance/retraction and constantly giving the second output shaft an amount of advance/retraction of a constant ratio (≠1) with respect to the amount of advance/retraction given to the first output shaft.

## Description

### TECHNICAL FIELD

The present invention relates to a positioning unit for changing a placement angle of a movable member and positioning a distal end thereof in a predetermined position, the positioning unit being used for, for example, holding a surgical instrument for a surgical operation using an endoscope and to position a distal end of the surgical instrument inserted into a human body relative to a treatment region.

### BACKGROUND ART

In recent years, as a medical surgical operation to a human body, there has been widely performed a so-called endoscopic operation in which a video camera called an endoscope is inserted into the body and an operation is performed with the help of a video of the inside of the body displayed through the endoscope without opening the abdomen. In the endoscopic operation, for example, several holes of several cm are provided in the abdomen of a patient. Treatment instruments such as the endoscope, a clamp, and a laser knife are inserted into the abdominal cavity through the holes and a trouble region in the abdominal cavity is surgically treated. According to the endoscopic operation, damage to the human body is small and the patient can be rehabilitated as early as possible after the operation, as compared with the case where surgical treatment is performed by opening the abdomen of the patient. Therefore, the endoscopic operation has been rapidly becoming widespread in recent years.

When such endoscopic operation is to be performed, it is necessary to use a surgical instrument which can be inserted into the human body through a hole of several cm and by which treatment within the human body can be performed instead of by the surgeon's hands. Up to now, as this kind of operation instrument, there has been known a surgical instrument as disclosed in JP 07-194608 A. Arm portions which can be opened and closed are provided at the distal end of a pipe portion inserted into the body. When the arm portions are opened and closed in the body, the tissues of the body are held and pulled for treatment. An operation manipulator constructed such that the distal end of a pipe portion inserted into the body is bent back and forth like the human wrist has been disclosed in JP 07-136173 A.

On the other hand, in the endoscopic operation, an affected treatment area is searched using the endoscope inserted into the human body. Then, the surgical instrument such as the laser knife or the clamp, which is inserted into the human body is approached to the affected treatment area. Therefore, it is necessary to move the distal end of the surgical instrument vertically and horizontally within the human body. However, in the endoscopic operation, the surgical instrument is inserted through the hole of several cm which is formed by cutting the skin and the muscle of a patient, with result that the hole is not allowed to expand. Therefore, when the distal end of the surgical instrument is to be operated in the human body to treat the affected treatment area, it is necessary to change a placement angle of the surgical instrument about the hole formed in skin tissues to move the distal end of the surgical instrument up and down and sideways.

As compared with the normal operation performed by opening the abdomen, a doctor that performs the endoscopic operation must have some skill, so that it is hard to perform the endoscopic operation by a doctor other than a specialist. For the patient, this means he or she can receive can receive the endoscopic operation only in limited number of hospitals that employ the specialist. However, the endoscopic operation is an operation which is performed by manipulating the surgical instrument while observing a video of the inside of the body displayed on a monitor. The video obtained by the endoscope can be transmitted to a distant place through a telephone line. Therefore, if the surgical instrument can be suitably moved by remote control, this proves useful because a specialist at a distant location can also perform the endoscopic operation and it is possible to select the endoscopic operation from treatment methods even in a local provincial city or the like in which the specialist does not exist.

### DISCLOSURE OF THE INVENTION

The present invention has been made in view of such problems. An object of the present invention is to provide a positioning unit which can position a distal end of a surgical instrument in an area to be treated in a human body without expanding a hole formed in the body to insert the surgical instrument, for example, in a surgical operation using an endoscope, and which can realize such positioning operation of the surgical instrument by remote manipulation.

To attain the above object, a positioning unit according to the present invention includes first and second output shafts whose distal ends are pivotably connected with a movable member of a surgical instrument or the like and which are provided parallel to each other and capable of reciprocating; and drive means for giving an arbitrary amount of advance/retraction to the first output shaft and constantly giving to the second output shaft an amount of advance/retraction at a constant ratio (≠1) with respect to the amount of advance/retraction given to the first output shaft.

When the movable member is pivotably connected with the distal ends of the first output shaft and the second output shaft which are arranged parallel to each other to thereby construct a link mechanism and different mounts of advance/retraction are given to the first and second output shafts, the placement angle of the movable member is changed according to the amounts of advance/retraction, so that the distal end of the movable member can be positioned. At this time, when an amount of advance/retraction at a constant ratio (≠1) with respect to the amount of advance/retraction given to the first output shaft is to be constantly given to the second output shaft, the placement angle of the movable member is always changed about a point due to the triangular similitude principle. Therefore, in the case where the surgical instrument such as a clamp is used as the movable member, when, in holding the surgical instrument by the positioning unit of the present invention, a center point for changing the placement angle is aligned with an insertion opening formed in the human body by cutting, even when the placement angle of the surgical instrument changes, the surgical instrument does not expand the insertion opening, so that the distal end of the movable member can be positioned in the area to be treated in the body. Thus, it is possible to handle the surgical instrument without using the hand of an operator, with the result that the positioning operation of the surgical instrument can be also realized by remote manipulation.

The positioning unit of the present invention successively changes the placement angle of the movable member in one direction, so that the distal end of the movable member moves only in a line. When two positioning units are connected in series with each other to construct a positioning arm, the end of the movable member can be vertically and horizontally moved.

### BRIEF DESCRIPTION OF THE DRAWINGS

Fig. 1 is a schematic view showing how an endoscopic operation is performed using a positioning unit of the present invention;
Fig. 2 is a perspective view showing an outward appearance of the positioning unit to which the present invention is applied;
Fig. 3 is a perspective view showing an internal structure of the positioning unit shown in Fig. 2;
Fig. 4 is a perspective view showing another example of a connection portion according to an embodiment of the present invention;
Fig. 5 is a sectional view showing an internal structure of the positioning unit shown in Fig. 2;
Fig. 6 shows a relationship between an advance and retraction ratio between first and second output shafts and a placement angle of a surgical instrument;
Fig. 7 is a schematic view showing an output shaft advance and retraction mechanism using racks and pinion gears;
Fig. 8 is a plan view showing a connection state between a first unit and a second unit;
Fig. 9 shows a schematic structure of a part ejection device using the positioning unit of the present invention; and
Fig. 10 is a schematic view showing an operating state of the part ejection device shown in Fig. 9.

### [Description of Symbols]

10 ... surgical instrument, 11 ... rod, 20 ... first unit, 21 ... first output shaft, 22 ... second output shaft, 21a, 22a ... external thread groove, 23 ... casing, 24 ... first ball thread nut, 25 ... second ball thread nut, 30 ... second unit, 50 ... hollow motor, P ... patient

### BEST MODE FOR CARRYING OUT THE INVENTION

Hereinafter, a positioning unit of the present invention will be described in detail with reference to the accompanying drawings.

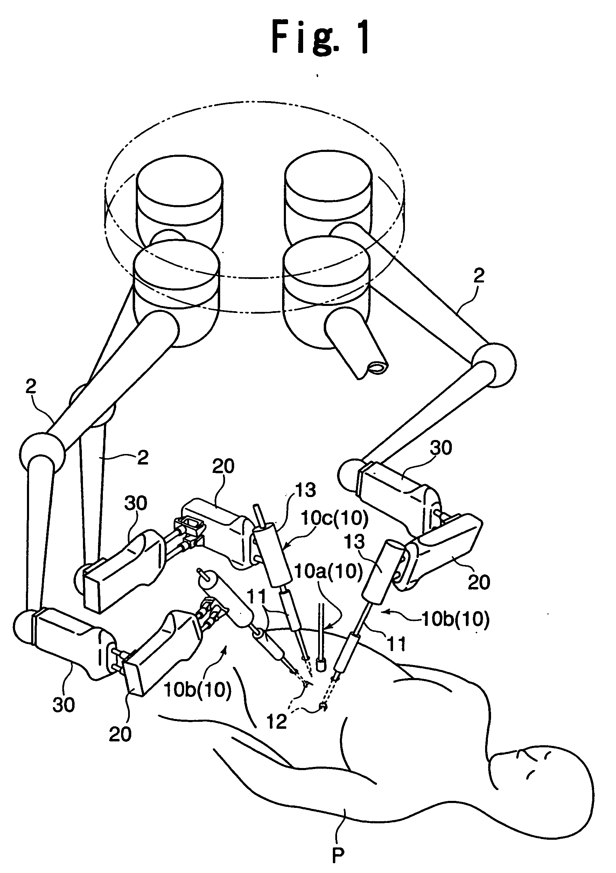

Fig. 1 shows a state in which surgical instruments such as a clamp and a laser knife are held using a positioning unit of the present invention and an endoscopic operation is performed while the surgical instruments are manipulated by the positioning unit. In the endoscopic operation, the skin of a patient P to be operated on is cut at reversal locations by about 2 cm to 3 cm. Distal ends of surgical instruments 10 such as an endoscope 10a serving as a video scope, a clamp 10b, and a laser knife 10c are inserted into a human body from respective insertion openings formed by the cutting. An operator observes a video of an affected treatment area in the human body, which is picked up by the endoscope 10a and displayed on a television monitor. The operator manipulates the other surgical instruments 10b, 10c based on a result obtained by the observation to perform suitable treatment such as clipping of the affected treatment area, holding thereof, or removal thereof.

In the example shown in Fig. 1, the respective surgical instruments 10 are provided with rods 11 inserted into the human body. A mechanism portion 12 suited for the application of each of the surgical instruments 10 is provided at a distal end of each of the rods 11. A drive portion 13 for moving the mechanism portion 12 is provided at a rear end of each of the rods 11.

On the other hand, the respective surgical instruments 10 are held by the positioning unit to which the present invention is applied. A casing housing the drive portion 13 for each of the surgical instruments 10 is connected with a first unit 20. The first unit 20 is connected with a second unit 30. The first and second units 20, 30 compose a positioning arm 1 for moving the surgical instrument 10 up and down and sideways. The second unit 30 is supported by a movable arm 2 fixed to a ceiling. Therefore, the first unit 20 and the second unit 30 can be fixed at a predetermined height and a predetermined position relative to the patient P.

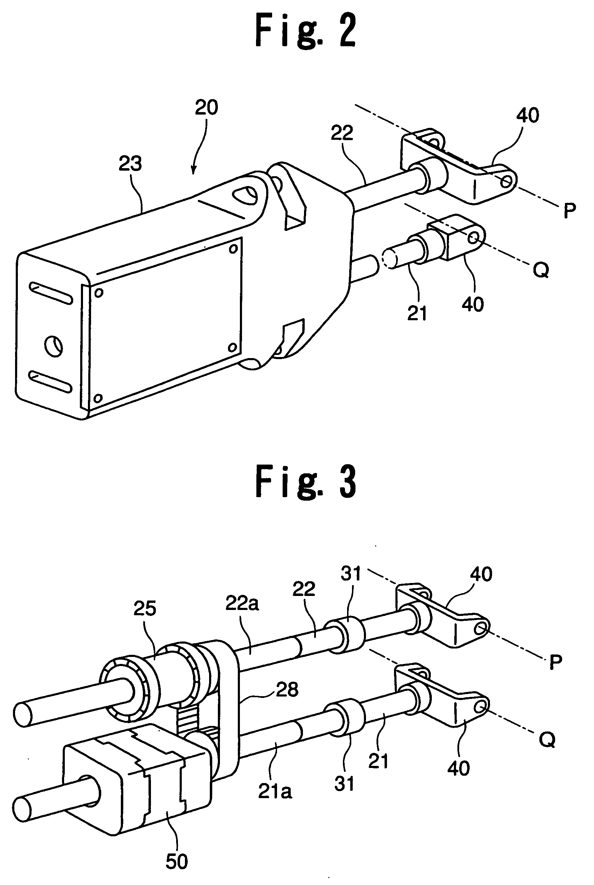

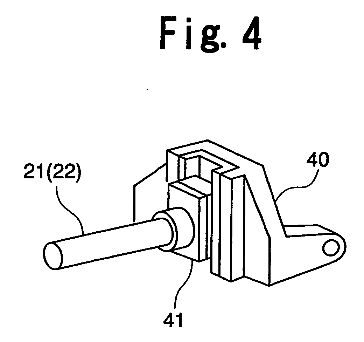

Fig. 2 shows an outline of the first unit 20 and Fig. 3 shows an internal structure thereof. The positioning unit 20 includes a first output shaft 21 and a second output shaft 22 which are provided parallel to each other. The respective output shafts 21, 22 are advanced from or retracted into a casing 23 in conjunction with eachother. Aconnectionportion 40 rotatablyconnectedwith a casing of the surgical instrument 10 is provided at a distal end of each of the respective output shafts 21, 22, so that the surgical instrument 10 is supported to be rockable about axes P and Q indicated by dashed lines. Note that the connection portion 40 may be the one provided at the distal end of the first output shaft 21 as shown in Fig. 2 or a relatively compact one provided at the distal end of the second output shaft 22 as shown in the drawing. When the first output shaft 21 and the second output shaft 22 are advanced or retracted to provide a suitable placement angle to the surgical instrument 10, a distance L between the rotational axis P for the distal end of the first output shaft 21 and the rotational axis Q for the distal end of the second output shaft 22 varies. When this is taken into consideration, as shown in Fig. 4, the connection portion may be provided through a linear bearing 41 absorbing the variation.

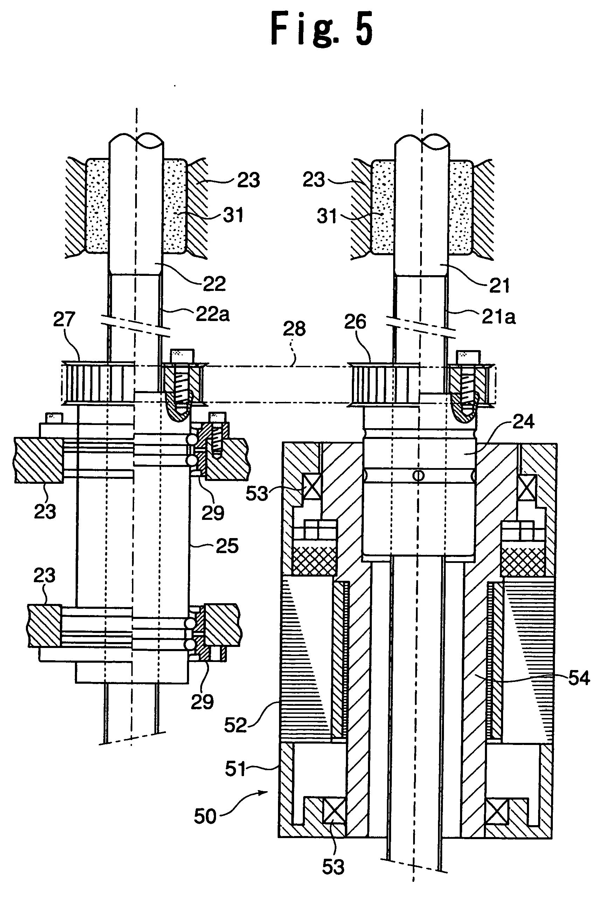

As shown in Fig. 3, spiral external thread grooves 21a, 22a are formed in the first output shaft 21 and the second output shaft 22. A first ball thread nut 24 and a second ball thread nut 25 are threaded into the external thread groove 21a of the first output shaft 21 and the external thread groove 22a of the second output shaft 22, respectively, through a large number of balls which are endlessly circulated. As shown in a sectional view of Fig. 5, the first ball thread nut 24 threaded onto the first output shaft 21 is stored in a hollow motor 50. The hollow motor 50 is composed of a motor housing 51 fixed to the casing 23 of the positioning unit 20, a motor stator 52 fixed to the motor housing 51, and a substantially cylindrical motor rotor 54 which is rotatably supported to the motor housing 51 through a bearing 53. The first output shaft 21 extends through a hollow portion of the motor rotor 54 and the first ball thread nut 24 is in fit engagement with an inner surface of the motor rotor 54. Therefore, when the motor stator 52 is excited to rotate the motor rotor 54, the first ball thread nut 24 fixed to the motor rotor 54 rotates. The distal end of the first output shaft 21 is fixed to the surgical instrument 10, so that it cannot be rotated. Thus, when the first ball thread nut 24 rotates due to the rotation of the motor rotor 54, the first output shaft 21 advances or retracts according to the speed of rotation.

On the other hand, a separate motor is not provided for the second ball thread nut 25. The second ball thread nut 25 is rotated by power transmitted through the first ball thread nut 24. A pulley 26 is fixed to an end of the first ball thread nut 24. A pulley 27 is fixed to an end of the second ball thread nut 25. A timing belt 28 is wound around the pulleys 26, 27. Therefore, when the first ball thread nut 24 is rotated by the hollow motor 50, the second ball thread nut 25 rotates at the same speed of rotation. The distal end of the second output shaft 22 is fixed to the surgical instrument 10, so that it cannot be rotated. Thus, when the second ball thread nut 25 rotates, the second output shaft 22 advances or retracts according to the speed of rotation. Note that the second ball thread nut 25 is rotatably supported to the casing 23 of the positioning unit through a support bearing 29. As shown in Figs. 3 and 5, journal portions of the first output shaft 21 and the second output shaft 22 are rotatably supported to the casing 23 of the positioning unit 20 by bearing sleeves 31 each made of resin.

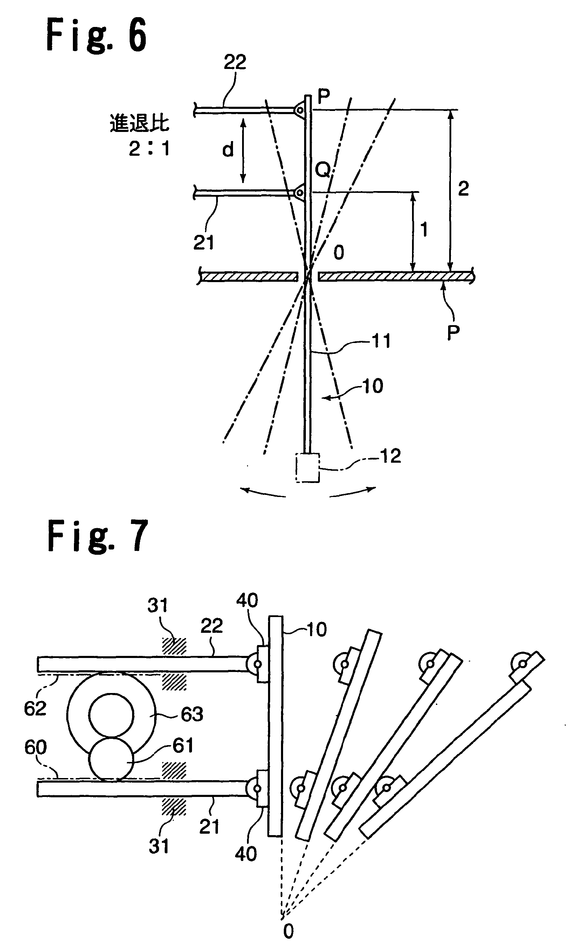

In this embodiment, a lead of the spiral external thread groove 22a formed in the second output shaft 22 is twice longer than that of the spiral external thread groove 22a formed in the first output shaft 21. Therefore, when the first output shaft 21 is advanced or retracted due to the rotation of the hollow motor 50, the second output shaft 22 is constantly advanced or retracted by the amount of advance/retraction which is twice larger than that of the first output shaft 21. Thus, as shown in Fig. 6, a placement angle of the surgical instrument 10 pivotably attached to the distal ends of the first output shaft 21 and the second output shaft 22 is changed, with the result that the mechanism portion 12 located at the distal end of the surgical instrument 10 can be positioned in the affected treatment area.

At this time, the rod 11 of the surgical instrument 10 rotates about a point to change the placement angle. Such a center point O is produced in a position corresponding to a ratio between the amount of advance/retraction of the first output shaft 21 and that of the second output shaft 22. As described in this embodiment, in the case where an advance/retraction ratio between the first output shaft 21 and the second output shaft 22 is 1:2, when an interval between the first output shaft 21 and the second output shaft 22 is d, the center point O is produced at a position at a distance d from a pivot axis Q connecting the first output shaft 21 with the surgical instrument 10 (see Fig. 6). This is based on a triangular similitude principle. When the advance/retraction ratio is 1:2, the placement angle of the rod 11 of the surgical instrument 10 is changed about the point O where a ratio between a line segment OQ and a line segment OP becomes 1:2. Therefore, when the advance/retraction ratio between the first output shaft 21 and the second output shaft 22 is constant at all times, the point O which becomes the center of change in placement angle of the rod 11 always exists.

When the advance/retraction ratio between the first output shaft 21 and the second output shaft 22 is to be made constant, as described above, the lead of the external thread groove 21a of the first output shaft 21 may be made different from the lead of the external thread groove 22a of the second output shaft 22. The same lead may be used to provide a constant ratio between the speed of rotation of the first ball thread nut 24 and the speed of rotation of the second ball thread nut 25. When the number of teeth formed in the pulleys are made different from each other in the case where torque is to be transmitted from the first ball thread nut to the second ball thread nut through the timing belt, the above-mentioned rotation speed ratio can be easily provided.

Assume that a height of the first unit 20 is adjusted using the movable arm 2 such that the center point O is aligned with an insertion opening formed in the patient P by cutting. Here, even when the placement angle of the surgical instrument 10 is changed by the first unit 20, the rod 11 of the surgical instrument 10 does not expand the insertion opening formed in the patient P. Therefore, the mechanism portion 12 located at the distal end of the surgical instrument 10 can be positioned with respect to the affected treatment area of the human body, without damaging skin tissues and the like near the insertion opening.

Note that a structure for advancing and retracting the first output shaft 21 and the second output shaft 22 in the first unit 20 is not limited to a combination of the ball thread nut and the external thread groove as described above. For example, as shown in Fig. 7, a combination of a rack and a pinion gear which are formed in each of the output shafts 21, 22 may be used. In this case, a first pinion gear 61 engaged with a rack 60 formed in the first output shaft 21 is provided and a second pinion gear 63 engaged with a rack 62 formed in the second output shaft 22 is provided. Any one of the pinion gears is rotated by a motor and torque caused by the one pinion gear is transmitted to the other pinion gear. The advance/retraction ratio between the first output shaft 21 and the second output shaft 22 may be set such that the speed of rotation of the first pinion gear 61 is made equal to that of the second pinion gear 63 and reference pitches of the racks 60, 62 formed in the respective output shafts 21, 22 are made different from each other or such that the reference pitches of the racks 60, 62 are made equal to each other and the speed of rotation of the first pinion gear 61 is made different from that of the second pinion gear 63.

In the example of the first unit 20 shown in Figs. 3 and 5, the hollow motor 50 is provided for only the first ball thread nut 24 and the power of the hollow motor 50 is transmitted to the second ball thread nut 25. However, a separate motor may be provided for the second ball thread nut 25. In this case, it is necessary to control the motor for driving the first ball thread nut 24 and the motor for driving the second ball thread nut 25 such that a ratio of amount of advance/retraction between the first output shaft 21 and the second output shaft 22 and a ratio of advance/retraction speed therebetween are constant at all times.

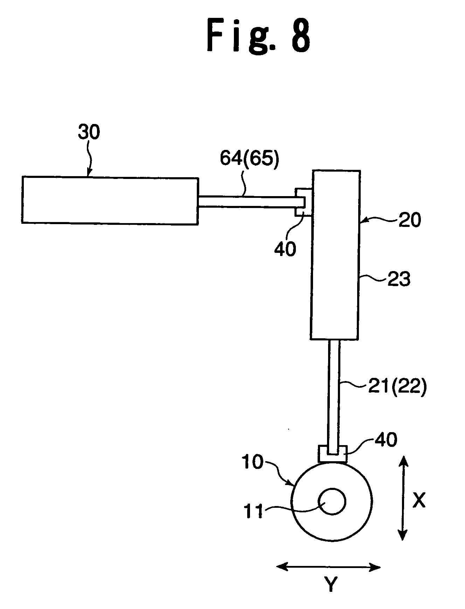

On the other hand, the second unit 30 has completely the same structure as the first unit 20 and is constructed such that a third output shaft 64 and a fourth output shaft 65 which are arranged parallel to each other 22 can be advanced or retracted at a predetermined advance/retraction ratio. Fig. 8 is a plan view showing a connection state among the surgical instrument, the first unit, and the second unit. As shown in this figure, the respective output shafts 64, 65 of the second unit 30 are pivotably coupled to the casing of the first unit 20 through the connection portion 40. In such a coupling state, the respective output shafts 64, 65 of the second unit 30 are always orthogonal to the respective output shafts 21, 22 of the first unit 20. That is, a plane including the first output shaft 21 and the second output shaft 22 of the first unit 20 is always orthogonal to a plane including the third output shaft 64 and the fourth output shaft 65 of the second unit 30. When the respective output shafts 21, 22 of the first unit 20 are advanced or retracted, the placement angle of the rod 11 of the surgical instrument 10 is changed along an X-direction indicated by an arrow line in Fig. 8. However, when the respective output shafts 64, 65 of the second unit 30 are advanced or retracted in the above-mentioned coupling state, the placement angle of the rod 11 of the surgical instrument 10 is changed along a Y-direction orthogonal to the X-direction. Therefore, when the first unit and the second unit are coupled to each other to construct a positioning arm as shown in Fig. 8, the rod of the surgical instrument held by the first unit can be moved in the X-direction and the Y-direction. Thus, it is possible to vertically and horizontally position the mechanism portion provided at the distal end of the rod in the human body.

In other words, when an arbitrary amount of rotation is imparted to each of the motors of the first unit and the second unit, the mechanism portion which is located at the distal end of the surgical instrument and inserted into the human body can be freely positioned with respect to the affected treatment area. When the motors of the first unit and the second unit are controlled while observing a video taken by the endoscope, a specialist at a distant location can also perform the endoscopic operation.

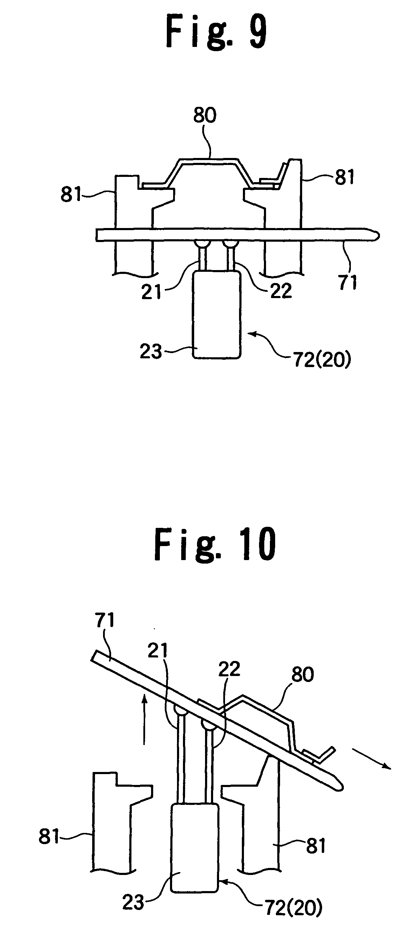

Figs. 9 and 10 show an example in which the positioning unit of the present invention is applied to another application, more specifically, a part ejection device for taking a machined mechanical part 80 such as a body part of an automobile from a jig 81 and ejecting it.

A part ejection device 70 includes a chute 71 serving as a movable member, which is formed as a plate whose surface is smooth and lifts the machined mechanical part up from below, and an actuator unit 72 for changing a placement angle of the chute 71, with one end of the chute 71 taken as a rocking center. A unit identical to the first unit 20 shown in Fig. 2 is used as the actuator unit 72. The distal end of the first output shift 21 and the distal end of the second output shaft 22 are pivotably supported to a rear surface of the chute 71. The mechanical part 80 is machined in a state in which it is positioned by the jig 81. During this machining, the chute 71 is set in a stand-by position shown in Fig. 9, that is, a position located below the mechanical part 80. After the completion of machining, to take out and eject the mechanical part 80 from the jig 81, the hollow motor 50 incorporated in the actuator unit 72 starts to rotate and then the first output shaft 21 and the second output shift 22 are advanced from the casing 23. Therefore, as shown in Fig. 10, the chute 71 is lifted upward to take out the processed mechanical part 80 from the jig 81. In addition, when the hollow motor 50 rotates, the amount of advance of the first output shaft 21 is different from that of the second output shift 22. Therefore, the placement angle of the chute 71 changes at the same time it is lifted upward, so that the mechanical part 80 is ejected from the jig so as to slide down the chute 71.

At this time, when the positioning unit of the present invention is used as the actuator unit 72, the placement angle of the chute 71 can be changed with its one end taken as the rocking center. When a carrying device such as a belt conveyer is provided adjacent to the chute 71 set in the stand-by position, the mechanical part 80 ejected from the jig 81 so as to slide down the chute 71 can be smoothly transferred to the carrying device. Thus, the positioning unit of the present invention is also useful when applied to various actuators in industrial robots and the like.

### INDUSTRIAL APPLICABILITY

As described above, according to the positioning unit of the present invention, the movable member is pivotably connected with the distal ends of the first output shaft and the second output shaft which are arranged parallel to each other to thereby construct a link mechanism. The ratio of amount of advance/retraction between the first output shaft and the second output shaft are maintained constant so that the placement angle of the movable member always changes about one point. Therefore, for example, in the case where the surgical instrument is held as the movable member, even when the placement angle of the surgical instrument changes, the surgical instrument does not expand the insertion opening formed in the human body by cutting. Thus, the distal end of the surgical instrument can be positioned with respect to an area to be treated in the body by remote manipulation.

## Claims

1. A positioning unit that holds a movable member and changes a placement angle of the movable member to perform positioning of a distal end of the movable member, **characterized by** comprising:
first and second output shafts whose distal ends are pivotably connected with the movable member and which are provided parallel to each other and capable of reciprocating; and drive means for giving an arbitrary amount of advance/retraction to the first output shaft and constantly giving to the second output shaft an amount of advance/retraction at a constant ratio (≠1) with respect to the amount of advance/retraction given to the first output shaft.

2. A positioning unit according to claim 1, **characterized in that** the drive means comprises external threads respectively formed in the first and second output shafts, a first nut and a second nut into which the external threads are respectively threaded, and a motor for rotating the first nut and the second nut.

3. A positioning unit according to claim 2, **characterized in that** a lead of the external thread formed in the first output shaft is different from a lead of the external thread formed in the second output shaft.

4. A positioning unit according to claim 2, **characterized in that** a speed of rotation of the first nut is different from a speed of rotation of the second nut.

5. A positioning unit according to claim 1, **characterized in that** the drive means comprises racks respectively formed in the first and second output shafts, a first pinion and a second pinion which are respectively engaged with the racks, and a motor for rotating the first pinion and the second pinion.

6. A positioning unit according to claim 5, **characterized in that** a reference pitch of the rack formed in the first output shaft is different from a reference pitch of the rack formed in the second output shaft.

7. A positioning unit according to claim 5, **characterized in that** a speed of rotation of the first pinion is different from a speed of rotation of the second pinion.

8. A positioning unit according to any one of claims 1 to 7, **characterized in that** the movable member is a surgical instrument for medical care and that a distal end of the surgical instrument is positioned with respect to an area to be treated.

9. A positioning arm that holds a movable member and changes a placement angle of the movable member to perform positioning of a distal end of the movable member, **characterized by** comprising:
a first unit that holds the movable member; and
a second unit that holds the first unit,
the positioning arm being **characterized in that**:
the first unit comprises: first and second output shafts whose distal ends are pivotably connected with the movable member and which are provided parallel to each other and capable of resiprocating; drive means for giving an arbitrary amount of advance/retraction given to the first output shaft and constantly giving to the second output shaft an amount of advance/retraction at a constant ratio (≠1) with respect to the amount of advance/retraction given to the first output shaft; and a casing that houses the drive means; and
the second unit comprises: third and fourth output shafts whose distal ends are pivotably connected with the casing of the first unit and which are provided parallel to each other and capable of reciprocating, the third and fourth output shafts being respectively orthogonal to the first and second output shafts of the first unit; and drive means for giving an arbitrary amount of advance/retraction to the third output shaft and constantly giving to the fourth output shaft an amount of advance/retraction at a constant ratio (≠1) with respect to the amount of advance/retraction given to the third output shaft.

10. A positioning arm according to any one of claims 1 to 7, **characterized in that** the movable member is a surgical instrument for medical care and that a distal end of the surgical instrument is positioned with respect to an area to be treated.
